# EUROPEAN PATENT SPECIFICATION

(11) **EP 2 340 867 B1**
(45) Date of publication and mention of the grant of the patent: **15.05.2013**
(21) Application number: 09425541.1
(22) Date of filing: 30.12.2009
(51) Int. Cl.: A61M 16/16, G01F 23/68

(54) **Humidification chamber with a safety sensor for detecting excessive levels of liquid**
Befeuchtungskammer mit Sicherheitssensor zur Erkennung eines zu hohen Füllstandes von Flüssigkeiten
Une chambre d'humidification avec un capteur de sécurité pour détecter un niveau excessif de liquide

(43) Date of publication of application: 06.07.2011
(73) Proprietor: Deas S.R.L., 48014 Castel Bolognese RA (IT)
(72) Inventor: Scardovi, Domenico, 48014 Castel Bolognese (Ravenna) (IT)
(74) Representative: Modiano, Micaela Nadia

(56) References cited:
- EP-A1- 2 119 466
- EP-A2- 1 366 881
- WO-A1-01/67054
- DE-A1- 3 905 745
- DE-A1- 3 939 496
- GB-A- 239 903
- GB-A- 811 417
- US-A- 1 756 053
- US-A- 4 351 731
- US-A1- 2006 151 624
- US-A1- 2007 101 811

## Description

The present invention relates to a humidification chamber of assisted respiration units provided with a safety sensor, for detecting excessive levels of liquid.

If a patient requires respiratory support by means of ventilators, adequately humidified air is supplied to him for allowing easier inhaling thereof.

For this purpose, it is known to use humidifiers provided with a heat source capable of heating the (generally metallic) base of a humidification chamber, so as to cause the evaporation of the water introduced therein: the resulting water vapor is mixed with the air, which can thus be passed on to the patient.

Typically, the water introduced in the chamber originates from a bag or a bottle which are arranged for example one meter above the chamber; from there, simply by gravity the water descends inside a tube until it reaches the chamber.

A particularly critical aspect of these humidification devices is control of the level of the water that is present in the chamber. It is in fact necessary to eliminate the risk of exceeding a set maximum level, beyond which there would be the risk of passing water in the liquid state to the patient, with consequent danger of drowning/suffocation.

To prevent this possibility, humidifiers are commercially widespread which are provided with a float arranged inside the chamber, which closes the water delivery hole once the liquid has reached a given level.

The presence of a single float inside the chamber, however, does not offer sufficient guarantee of protection for the patient: a malfunction of such float, for example due to a deformation of the coupled elements, which causes an undesired blockage of the delivery hole, or due to sticking of the float on the bottom of the chamber, which does not allow it to move to close the delivery hole, is in fact not infrequent.

It is possible to adopt two floats, both articulated to the walls of the humidification chamber: a respective element that will make it possible to prevent the introduction of water into the chamber as a consequence of the rise of the water level must be articulated to each float.

In particular, the rise of the water level causes the first element articulated to the first float to block the water delivery hole with its appropriately shaped top.

If a malfunction occurs, the further rise of the water level causes the second element, which is articulated to the second float, to block a second hole, which connects the humidification chamber, connected to the patient, to a pre-chamber along the walls of which the delivery hole is provided.

Therefore, in case of malfunction of the first float, the second float, which floats less than the first one, can be activated in order to prevent the passing of water to the patient, allowing only the pre-chamber to fill.

However, even this solution is not free from drawbacks.

As is evident from the description given above, this is a solution which has a high complexity, due to the various articulations that are present and to the numerous elements that are needed to interconnect the walls, the floats and the elements assigned to close the above cited holes.

Each float must in fact be connected to the walls of the chamber and to the element assigned to closure of the water inlet holes by means of suitable guiding elements and hinges suitable to provide the desired articulations.

The presence of such a large number of elements therefore makes it complicated to provide and/or assemble the humidifier; further, in order to ensure the good operation of the device, it is necessary to resort to materials that have high-level mechanical characteristics, with a consequent increase in costs.

It should be added to the above that the presence of these complex articulations in any case makes the humidifier clearly susceptible to breakages, with consequent loss of reliability of the device, especially following frequent and repeated use.

Moreover, it is important to note that as a consequence of a malfunction of the first float a filling, albeit a partial one, of the humidifier occurs; the water can in fact enter through the delivery hole and can fill the cavity formed by the pre-chamber. If the walls of such pre-chamber deteriorate or in any case are damaged, even slightly, this leads to an undesired outward dripping of water.

Finally, it should be noted that in the described solution the drag offered to the air stream inside the chamber is particularly high due to the large number of elements that are present and due to their shape.

Moreover, the inadequacy of an apparatus that comprises two safety elements (floats and respective articulations) whose operation is based on the same physical principle of mechanical blockage of a hole is to be noted: a condition which is difficult for a first element is therefore difficult also for the second element, which works in a similar manner.

Adjustment of the inflow of water, moreover, is not permitted, but only a change of state from "open duct " to "closed duct" is allowed.

EP 2 119 466 discloses a humidifier with a two-float safety system for controlling the liquid level in a containment chamber.

GB 811 417 and GB 239 903 disclose liquid level indicators usable for remote indication of the liquid level in a drum or a tank or a receptacle.

The aim of the present invention is to solve the problems described above, by providing a safety sensor for detecting excessive levels of liquid in a humidification chamber of assisted respiration units which ensures that the chamber has a pair of independent safety elements which apply different physical phenomena to control the incoming water flow.

Within this aim, an object of the invention is to propose a safety sensor for detecting excessive levels of liquid in a humidification chamber of assisted respiration units which is capable of adjusting the extent of the water flow in input.

Further object of the invention is to propose a humidification chamber with a safety sensor for detecting excessive levels of liquid in a humidification chamber which can generate a signal that is proportional to the level reached by the water in the respective chamber.

Another object of the invention is to propose a humidification chamber provided with a safety sensor in which the water level inside it is managed by means of a feedback control.

A further object of the present invention is to provide a humidification chamber with a safety sensor for detecting excessive levels of liquid in a humidification chamber of assisted respiration units and a humidification chamber provided with a safety sensor which has low costs, is relatively simple to provide in practice and is safe in application.

This aim and these objects are achieved by a humidification chamber, provided with a safety sensor for detecting excessive liquid levels, that has the features set forth in claim 1.

Further characteristics and advantages of the invention will become better apparent from the following detailed description of a preferred but not exclusive embodiment of the safety sensor for detecting excessive levels of liquid in a humidification chamber of assisted respiration units and of the humidification chamber provided with a safety sensor according to the invention, illustrated by way of non-limiting example in the accompanying drawings, wherein:

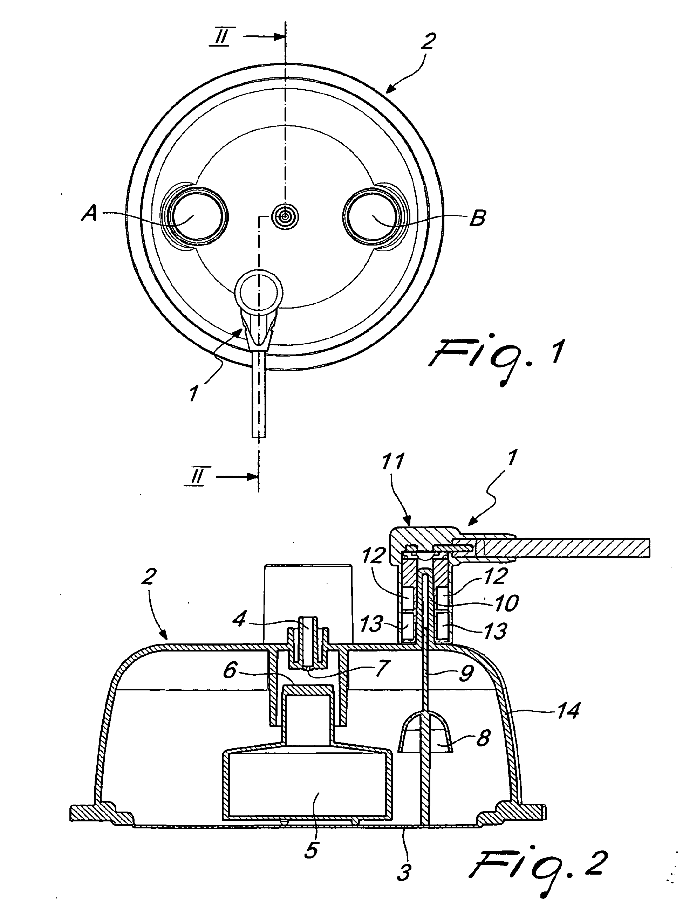

Figure 1 is a top view of the humidification chamber provided with a safety sensor according to the invention;
Figure 2 is a sectional view, taken along a substantially transverse plane that passes through the sensor, of the humidification chamber provided with a safety sensor according to the invention;

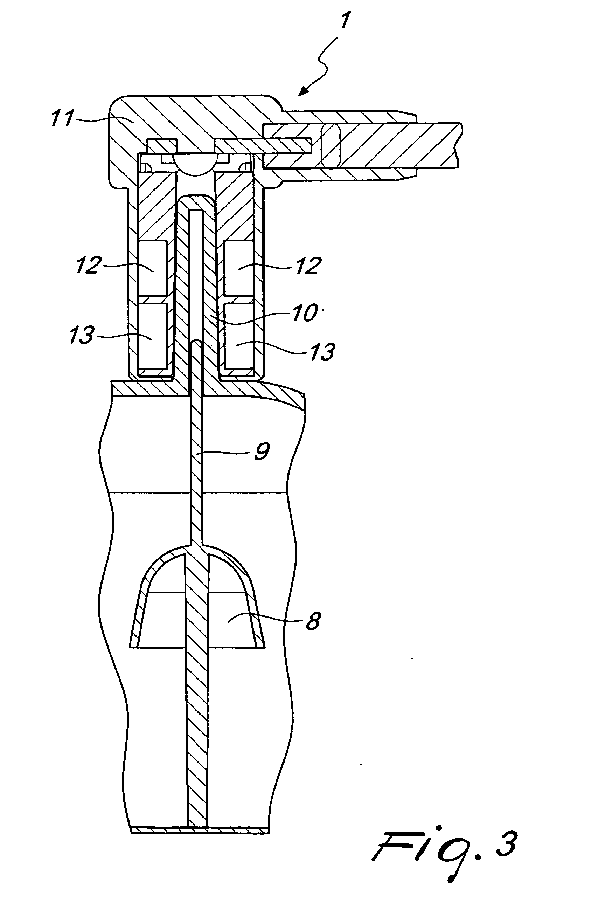

Figure 3 is a sectional view, taken along a transverse plane, of a sensor according to the invention.

With reference to the figures, the reference numeral 1 generally designates a safety sensor for detecting excessive levels of liquid in a humidification chamber 2 of assisted respiration units provided with a safety sensor 1.

Respiration units comprise an air conveyor and a heater on which the humidification chamber 2 is accommodated: the base 3 of the chamber 2 rests on a heated surface and the heat is thus transferred to the water contained within the chamber 2, which by evaporating ensures the correct addition of humidity to the conveyed air that passes through the chamber 2.

The air that reaches the patient is therefore humidified to the point of making it compliant with the therapeutic needs of the patient: the air stream enters for example through an opening A and exits from an outlet B.

Through a channel 4, the water is introduced into the chamber 2: the channel 4 generally constitutes the end portion of a duct which preferably arrives from a tank generally arranged upward with respect to the chamber 2 for exploiting gravity to convey the water toward the channel 4.

Generally, below the liquid intake channel 4 there is a first safety unit 5 designed to adjust the inflow of water into the chamber 2.

The first safety unit 5 consists of a float which, as the water level inside the chamber 2 increases, rises until its upper end 6 blocks a liquid inlet hole 7 which corresponds to the end of the channel 4.

The chambers 2 according to this embodiment are inherently safe, since they can adjust the inflow of liquid (water) into them directly by means of the action of the safety unit 5: if, however, the unit 5 fails (for example jams and becomes stuck due to misalignments and/or deformations of a thermal kind), the chamber 2 can fill with water completely. With this kind of malfunction, water might be introduced along the air channel and would reach the patient, causing severe damage and even suffocation.

It should also be specified that the adjustment performed by the first safety unit 5 is simply determined by the opening/closure of the hole 7: this, therefore, is not an actual adjustment of the flow-rate of fluid that circulates in the channel 4.

The safety sensor 1 according to the invention comprises a float 8, which is adapted to stay on the free surface of the liquid, generally water or aqueous solutions, contained in the chamber 2.

The float 8 is provided with an element which can slide on a guide 10.

For the sake of simplicity in description, reference shall be made hereinafter to a particular embodiment in which the element consists of a rod 9, which is arranged above the float 8, and a guide 10, which consists of a duct 10 whose shape is complementary to the shape of the rod 9 and which is therefore adapted to accommodate it partially and/or fully: of course, any other embodiment regarding the shape and characteristics of the element 9 and of the guide 10 is to be understood as comprised within the scope of the present invention.

Sliding is caused by the thrust applied by the liquid on the float 8: in the embodiment shown in the accompanying figures, the float 8 consists of an air pocket which is delimited by a shell; the possibility of using floating bodies of another kind as a function of the particular requirements of application is not excluded.

A signal circuit 11 is present along the duct 10 and can comprise the duct itself: the value in output from the signal circuit 11 is proportional to the length of the portion of rod 9 that is present in the duct 10.

According to a particular embodiment of unquestionable interest in practice and in application, the signal circuit 11 consists of a pair of coils 12, 13, i.e., components made of windings of electrical conductors.

In this case, the rod must be made of ferromagnetic material. The first coil 12 is supplied with electric power for the flow of an electric current therein: the magnetic flux that is generated will induce (due to the physical phenomenon of electromagnetic induction) the flow of an induced current on the second coil 13.

Such induced current will be proportional to the length of the portion of rod of ferromagnetic material that is present in the duct 10 which constitutes the magnetic coupling path. This is due to the fact that the magnetic coupling between two circuits improves as the magnetic permeability of the material that separates them increases: the use of a ferromagnetic material to provide the rod 9 guarantees an extremely high value of magnetic permeability when the rod is present and a very low permeability value when the duct 10 is empty. It is evident that the value of the current induced by the first coil 12 on the second coil 13 is proportional to the quantity of ferromagnetic material that is present in the duct 10 and therefore to the length of the portion of rod of ferromagnetic material that is present in the duct 10. According to a particular embodiment which is alternative to the preceding one, is particularly simple to provide and relatively simple to apply in practice, the signal circuit 11 can consist of a pair of plates of a capacitor which face each other.

In this case, the rod 9 is preferably made of dielectric material: the value of the electric capacitance detected between the two plates is proportional to the length of the portion of rod of dielectric material that is present in the duct 10. Of course, even if a non-dielectric material were used it would still be possible to obtain a significant variation of the value of electrical capacitance detected between the plates; therefore, the possibility of providing the rod 9 by using other materials is not excluded.

With reference to a further possible embodiment instead, the signal circuit 11 might comprise a light source and a light detector.

The detector (for example a photodiode or photocell of another kind) must be adapted to generate an electrical signal which is proportional to the intensity of the light beam that strikes it.

The rod 9 is therefore made of opaque and non-transparent material.

The length of the portion of rod 9 made of opaque, non-transparent material that is present in the duct 10, which is instead made of transparent material, constitutes a shield to the passage of light. The consequence is that the detector generates an electrical signal proportional to the position assumed by the rod 9 in the duct 10.

The three proposed embodiments are presented only by way of example: the possibility of providing signal circuits 11 based on other physical phenomena, according to an architecture which is entirely within the scope of the present invention, is in fact not excluded.

For example, the signal circuit 11 might comprise at least one switch arranged along the guiding duct 10: the state is changed by the tip of the rod 9 reaching the height where at least one corresponding switch is located.

In this manner, by means of a single switch it is possible to receive a signal that causes the opening/closure of the water delivery in the chamber 2: if a larger number of switches arranged at different heights is available, it is instead possible to adjust the delivery flow, since the level reached by the tip of the rod 9 will indicate the level of the water in the chamber 2 with enough precision to allow feedback control of the flow-rate of water to be introduced.

In each one of the listed cases (and also if different embodiments are adopted), the output terminals of the signal circuit 11 are connected to a control and management unit (not shown in the accompanying figures), which controls an actuator, preferably of the type of an electric valve or a controlled tap, which intercepts the liquid conveyance channel 4 and adjusts the flow of liquid through such channel. In this case, the control can also be provided in terms of flow-rate (i.e., by modifying the breadth of the opening of the channel, opening or closing the passage port of the electric valve, of the controlled tap or of another equivalent component) and not only by providing simple closure and opening of the hole 7.

It is not of secondary importance to point out that this flow-rate control is effectively proportional in each instant to the level of the liquid, since it is performed as a function of the position assumed progressively by the rod 9 in the duct 10 and therefore of the level of the liquid that is present within the chamber 2.

The operation of the entire assisted respiration unit is therefore optimized by adopting the sensor 1 according to the invention.

In particular, the control and management unit can even be associated with an indicator, preferably consisting of a display screen, which indicates the level reached by the liquid inside the chamber in which the sensor 1 is installed: this indication will allow an operator, even remotely, to check the correct operation of the chamber 2. Moreover, the association of an alarm which warns the operator that limit conditions have been reached which can indicate a malfunction of the entire component which determines an incorrect rise of the level of the liquid in the chamber 2 is not excluded.

The humidification chamber 2, provided with the safety sensor 1, generally consists of a dome 14 which is closed on the base 3 and is suitable for resting on a respective heater (as explained earlier).

The dome 14 must necessarily comprise a duct 10 for sliding accommodation of the rod 9.

The rod 9 (provided according to the specific indications of the type of sensor 1 that is intended to adopt) is always jointly connected, in a lower region, to the float 8, which is adapted to stay on the free surface of the liquid contained in the chamber 2. The rod 9, by sliding within the duct 10, interferes with the signal circuit 11 arranged outside the duct 10, incorporating it.

Generically, the signal circuit 11 must comprise a detector (of the type adapted to work in combination with the particular material of which the rod 9 is made) directly at the duct 10 and a control and management unit connected to the output terminals of such detector.

The control and management unit is functionally associated with an actuator designed to adjust the flow of liquid in the intake channel 4: the actuator is preferably of the type of an electric valve or a controlled tap.

Advantageously, the safety sensor 1 allows provision of a chamber 2 which has a pair of independent safety elements that apply different physical phenomena to control the inflow of water.

Conveniently, the safety sensor 1 is capable of adjusting the extent of the water inflow.

Positively, the sensor 1 is capable of generating a signal which is proportional to the level reached by the water in the respective chamber.

It should be noted that the humidification chamber 2 provided with safety sensor 1 guarantees that the level of the water inside it is managed by means of a feedback control.

It is important to specify that the sensor 1 is provided for the specific purpose of being associable with a respective chamber 2 and then disassembled from it and reassembled on another chamber 2.

This characteristic is fundamentally important, since by means of this type of sensor 1 it is possible to use chambers 2 of the disposable type, which however can ensure a level of safety for the patient that is extremely high due to the presence of the sensor 1, which performs a highly accurate control over the level of the liquid and preferably also allows a continuous adjustment of the flow of liquid introduced in the chamber 2.

The invention thus conceived is susceptible of numerous modifications and variations, all of which are within the scope of the appended claims; all the details may further be replaced with other technically equivalent elements.

In the exemplary embodiments shown, individual characteristics, given in relation to specific examples, may actually be interchanged with other different characteristics that exist in other exemplary embodiments.

In practice, the materials used, as well as the dimensions, may be any according to requirements and to the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A humidification chamber comprising a dome (14) which is closed on a base (3) suitable for resting on a respective heater, with consequent transfer of heat by conduction from the heater to the base (3) and accordingly to the liquid contained within said chamber (2), said chamber (2) comprising at least one channel (4) for introducing liquid, and an oscillating float (5) adapted to close a port (7) of the channel (4) that faces the dome (14) for introducing the liquid,
**characterized in that**
- said humidification chamber is provided with a safety sensor (1) for detecting excessive levels of liquid, said safety sensor (1) comprising a float (8) which is adapted to stay on the free surface of the liquid contained in said chamber (2);
- said dome (14) comprises a guide (10) for the sliding accommodation of an element (9), said element (9) being jointly associated in a lower region with the float (8)
- a signal circuit (11) is provided along said guide (10), the signal circuit (11) preferably comprising said guide (10), the value in output from said signal circuit (11) being defined by the length of the portion of the element (9) that is present on said guide (10);
- said signal circuit (11) comprises a detector directly at said guide (10), and a control and management unit which is connected to output terminals of said detector and is functionally associated with an actuator designed to adjust the flow of liquid in said intake channel (4).

2. The humidification chamber according to claim 1, **characterized in that** said element (9) is shaped substantially like an upper rod (9) and said guide (10) is shaped substantially like a duct (10), the value in output from said signal circuit (11) being proportional to the length of the portion of the rod (9) that is present in said duct (10).

3. The humidification chamber according to at least one of the preceding claims, **characterized in that** said signal circuit (11) consists of a pair of coils (12, 13), i.e., windings of electrical conductors, and said element (9), preferably rod (9) is made of ferromagnetic material, a first coil (12) being supplied electrically for the flow of an electric current therein, the magnetic flux that is generated inducing the flow of an induced current in the second coil (13), said induced current being proportional to the length of the portion of the element (9), preferably rod (9) made of ferromagnetic material that is present in said guide (10), preferably duct (10) forming the magnetic coupling path.

4. The humidification chamber according to at least one of claims 1 and 2, **characterized in that** said signal circuit (11) consists of a pair of plates of a capacitor which face each other and said element (9), preferably rod (9) is made of dielectric material, the value of the electrical capacitance that is detected between the two plates being proportional to the length of the portion of the element (9), preferably rod (9) made of dielectric material that is present in said guide (10), preferably duct (10).

5. The humidification chamber according to at least one of claims 1 and 2, **characterized in that** said signal circuit (11) comprises a light source and a light detector, which is adapted to generate an electrical signal that is proportional to the intensity of the light beam that strikes it, and said element (9), preferably rod (9) is made of opaque and nontransparent material, the length of the portion of the element (9), preferably rod (9) made of opaque and nontransparent material that is present in said guide (10), preferably duct made of transparent material, constituting a shield to the passage of light, said detector generating an electrical signal which is proportional to the position assumed by said element (9), preferably rod (9) in said guide (10), preferably duct (10).

6. The humidification chamber according to at least one of claims 1 and 2, **characterized in that** said signal circuit (11) comprises at least one switch which is arranged along said guide (10), preferably duct (10), its state being changed by the part of the tip of the element (9), preferably rod (9) reaching the height at which said at least one switch is arranged.

7. The humidification chamber according to claim 5, **characterized in that** said control and management unit is associated with an indicator, preferably consisting of a display screen, which indicates the level reached by the liquid inside the chamber (2) in which the sensor (1) is installed.

8. The humidification chamber according to any of the preceding claims, **characterized in that** an alarm is provided associated to said signal circuit (11) which warns the operator that limit conditions have been reached.

9. The humidification chamber according to any of the preceding claims, **characterized in that** said actuator is preferably of the type of an electric valve or a controlled tap.

## Patentansprüche

1. Eine Befeuchtungskammer, die eine Kuppel (14) umfasst, welche auf einer Basis (3) geschlossen ist, die geeignet ist, auf einer entsprechenden Heizvorrichtung aufzuliegen, mit daraus folgender Wärmeübertragung durch Leitung von der Heizvorrichtung zur Basis (3) und dementsprechend zu der Flüssigkeit, die in der Kammer (2) enthalten ist, wobei die Kammer (2) mindestens einen Kanal (4) zum Einführen von Flüssigkeit und einen schwingenden Schwimmkörper (5) umfasst, der ausgebildet ist, um eine Öffnung (7) des Kanals (4) zu schließen, die der Kuppel (14) zum Einführen der Flüssigkeit gegenüberliegt,
**dadurch gekennzeichnet, dass**
- die Befeuchtungskammer mit einem Sicherheitssensor (1) zur Erfassung überhöhter Flüssigkeitsstände versehen ist, wobei der Sicherheitssensor (1) einen Schwimmkörper (8) umfasst, der ausgebildet ist, um an der freien oberfläche der in der Kammer (2) enthaltenen Flüssigkeit zu bleiben,
- die Kuppel (14) eine Führung (10) zur verschiebbaren Aufnahme eines Elements (9) umfasst, wobei das Element (9) in einem unteren Bereich fest mit dem Schwimmkörper (8) verbunden ist,
- ein Signalkreis (11) entlang der Führung (10) bereitgestellt ist, wobei der Signalkreis (11) vorzugsweise die Führung (10) umfasst, wobei der Ausgabewert von dem Signalkreis (11) durch die Länge des Abschnitts des Elements (9) bestimmt wird, das an der Führung (10) vorhanden ist,
- der Signalkreis (11) direkt an der Führung (10) einen Detektor und eine Steuer- und Verwaltungseinheit umfasst, die mit Ausgangsklemmen des Detektors verbunden ist und funktionell mit einem Aktor verknüpft ist, der konstruiert ist, um die Strömung von Flüssigkeit in dem Einlasskanal (4) einzustellen.

2. Die Befeuchtungskammer gemäß Anspruch 1, **dadurch gekennzeichnet, dass** das Element (9) im Wesentlichen wie eine obere Stange (9) geformt ist und die Führung (10) im Wesentlichen wie ein Gang (10) geformt ist, wobei der Ausgabewert von dem Signalkreis (11) proportional zur Länge des Abschnitts der Stange (9) ist, der sich in dem Gang (10) befindet.

3. Die Befeuchtungskammer gemäß mindestens einem der obigen Ansprüche, **dadurch gekennzeichnet, dass** der Signalkreis (11) aus einem Paar von Spulen (12, 13), d. h. Windungen elektrischer Leiter, besteht und das Element (9), vorzugsweise die Stange (9), aus ferromagnetischem Material hergestellt ist, wobei eine erste Spule (12) für den Fluss eines elektrischen Stroms darin elektrisch versorgt wird, wobei der magnetische Fluss, der erzeugt wird, den Fluss eines induzierten Stroms in der zweiten Spule (13) induziert, wobei der induzierte Strom proportional zur Länge des Abschnitts des Elements (9), vorzugsweise der Stange (9), aus ferromagnetischem Material ist, das / die in der Führung (10), vorzugsweise dem Gang (10), vorhanden ist, die / der den Pfad der induktiven Kopplung bildet.

4. Die Befeuchtungskammer gemäß mindestens einem der Ansprüche 1 und 2, **dadurch gekennzeichnet, dass** der Signalkreis (11) aus einem Paar von Platten eines Kondensators besteht, die einander gegenüberliegen, und das Element (9), vorzugsweise die Stange (9), aus dielektrischem Material hergestellt ist, wobei der Wert der elektrischen Kapazität, die zwischen den zwei Platten gemessen wird, proportional ist zur Länge des Abschnitts des Elements (9), vorzugsweise der Stange (9), aus dielektrischem Material, das / die in der Führung (10), vorzugsweise dem Gang (10), vorhanden ist.

5. Die Befeuchtungskammer gemäß mindestens einem der Ansprüche 1 und 2, **dadurch gekennzeichnet, dass** der Signalkreis (11) eine Lichtquelle und einen Lichtdetektor umfasst, der ausgebildet ist, um ein elektrisches Signal zu erzeugen, das proportional zur Intensität des Lichtstrahls ist, der ihn trifft, und das Element (9), vorzugsweise die Stange (9), aus trübem und nicht transparentem Material hergestellt ist, wobei die Länge des Abschnitts des Elements (9), vorzugsweise der Stange (9), aus trübem und nicht transparentem Material, das / die in der Führung (10), vorzugsweise dem Gang (10), vorhanden ist, die / der aus transparentem Material hergestellt ist, eine Abschirmung gegen das Hindurchdringen von Licht bildet, wobei der Detektor ein elektrisches Signal erzeugt, das proportional zur Position ist, die von dem Element (9), vorzugsweise der Stange (9), in der Führung (10), vorzugsweise dem Gang (10), eingenommen wird.

6. Die Befeuchtungskammer gemäß mindestens einem der Ansprüche 1 und 2, **dadurch gekennzeichnet, dass** der Signalkrebs (11) mindestens einen Schalter umfasst, der entlang der Führung (10), vorzugsweise dem Gang (10), angeordnet ist, wobei sein Zustand durch den Teil der Spitze des Elements (9), vorzugsweise der Stange (9), geändert wird, der die Höhe erreicht, in welcher der mindestens eine Schalter angeordnet ist.

7. Die Befeuchtungskammer gemäß Anspruch 5, **dadurch gekennzeichnet, dass** die Steuerungs- und Verwaltungseinheit mit einer Anzeige verknüpft ist, die vorzugsweise in einer Bildschirmanzeige besteht, die den Stand anzeigt, der von der Flüssigkeit in der Kammer (2) erreicht wird, in welcher der Sensor (1) installiert ist.

8. Die Befeuchtungskammer gemäß einem beliebigen der obigen Ansprüche, **dadurch gekennzeichnet, dass** ein Alarm geliefert wird, der mit dem Signalkreis (11) gekoppelt ist und der den Bediener warnt, dass Grenzbedingungen erreicht wurden.

9. Die Befeuchtungskammer gemäß einem beliebigen der obigen Ansprüche, **dadurch gekennzeichnet, dass** der Aktor vorzugsweise von der Art eines Elektroventils oder eines gesteuerten Abgriffs ist.

## Revendications

1. Chambre d'humidification comprenant un dôme (14) qui est fermé au niveau d'une base (3) appropriée à reposer sur un dispositif de réchauffage respectif, par conséquent, un transfert de chaleur ayant lieu par conduction du dispositif de réchauffage à la base (3) et en fonction du liquide contenu dans ladite chambre (2), ladite chambre (2) comprenant au moins un canal (4) destiné à l'introduction de liquide et un flotteur oscillant (5) adapté à fermer une ouverture (7) du canal (4) faisant face au dôme (14) qui est destinée à l'introduction du liquide,
**caractérisée en ce que**
- ladite chambre d'humidification est munie d'un capteur de sécurité (1) destiné à la détection de niveaux excessifs de liquide, ledit capteur de sécurité (1) comprenant un flotteur (8) qui est adapté à rester sur la surface libre du liquide contenu dans ladite chambre (2),
- ledit dôme (14) comprend un dispositif de guidage (10) pour le logement coulissant d'un élément (9), ledit élément (9) étant associé conjointement au flotteur (8) dans une zone plus basse
- un circuit de signalisation (11) est prévu le long dudit dispositif de guidage (10), le circuit de signalisation (11) comprenant de préférence ledit dispositif de guidage (10), la grandeur de sortie dudit circuit de signalisation (11) étant définie par la longueur de la portion de l'élément (9) qui est présent sur ledit dispositif de guidage (10),
- ledit circuit de signalisation (11) comprend un capteur directement sur ledit dispositif de guidage (10) et une unité de commande et de gestion qui est reliée à des terminaux de sortie dudit capteur et associée fonctionnellement à un actionneur conçu pour ajuster l'écoulement de liquide dans ledit canal d'amenée (4).

2. Chambre d'humidification suivant la revendication 1, **caractérisée en ce que** ledit élément (9) est formé essentiellement comme une tige supérieure (9) et ledit dispositif de guidage (10) est formé essentiellement comme un conduit (10), la grandeur de sortie dudit circuit de signalisation (11) étant proportionnelle à la longueur de la portion de la tige (9) qui est présente dans ledit conduit (10).

3. Chambre d'humidification suivant au moins une des revendications précédentes, **caractérisée en ce que** ledit circuit de signalisation (11) consiste en une paire de bobines (12, 23), p. ex. des enroulements de conducteurs électriques et ledit élément (9), de préférence la tige (9), est réalisé dans une matière ferromagnétique, une première bobine (12) étant fournie électriquement pour le flux d'un courant électrique à l'intérieur, le flux magnétique qui est généré induisant le flux d'un courant induit dans la seconde bobine (13), ledit courant induit étant proportionnel à la longueur de la portion de l'élément (9), de préférence la tige (9), réalisé dans une matière ferromagnétique qui est présente dans ledit dispositif de guidage (10), de préférence, le conduit (10) formant le chemin de couplage magnétique.

4. Chambre d'humidification suivant au moins une des revendications 1 et 2, **caractérisée en ce que** ledit circuit de signalisation (11) est constitué d'une paire de plaques d'un condensateur qui se font face l'une à l'autre, et ledit élément (9), de préférence la tige (9), est réalisé dans une matière diélectrique, la valeur de capacité électrique qui est détectée entre les deux plaques étant proportionnelle à la longueur de la portion de l'élément (9), de préférence la tige (9) réalisé dans une matière diélectrique qui est présente dans ledit dispositif de guidage (10), de préférence le conduit (10).

5. Chambre d'humidification suivant au moins une des revendications 1 et 2, **caractérisée en ce que** ledit circuit de signalisation (11) comprend une source lumineuse et un détecteur de lumière qui est adapté à générer un signal électrique proportionnel à l'intensité du faisceau de lumière qui le frappe et ledit élément (9), de préférence la tige (9), est réalisé dans une matière opaque et non transparente, la longueur de la portion de l'élément (9), de préférence la tige (9), réalisé en une matière opaque et non transparente qui est présente dans ledit dispositif de guidage (10), de préférence le conduit (10), réalisé en une matière transparente, constituant un écran au passage de la lumière, ledit détecteur générant un signal électrique qui est proportionnel à la position adoptée par ledit élément (9), de préférence la tige (9), dans ledit dispositif de guidage (10), de préférence le conduit (10).

6. Chambre d'humidification suivant au moins une des revendications 1 et 2, **caractérisée en ce que** le circuit de signalisation (11) comprend au moins un interrupteur qui est disposé le long dudit dispositif de guidage (10), de préférence du conduit (10), son état étant changé par la partie de la pointe de l'élément (9), de préférence de la tige (9), atteignant la hauteur à laquelle ledit au moins un interrupteur est disposé.

7. Chambre d'humidification suivant la revendication 5, **caractérisée en ce que** ladite unité de commande et de gestion est associée à un voyant, consistant de préférence en un écran d'affichage qui affiche le niveau atteint par le liquide à l'intérieur de la chambre (2), dans laquelle le capteur (1) est installé.

8. Chambre d'humidification suivant une quelconque des revendications précédentes, **caractérisée en ce qu'**est prévue, associée audit circuit de signalisation, une alarme (11) qui avertit l'opérateur de ce que des conditions limites ont été atteintes.

9. Chambre d'humidification suivant une quelconque des revendications précédentes, **caractérisée en ce que** ledit actionneur appartient de préférence au genre d'une électrovanne ou d'un robinet commandé.
